# EUROPEAN PATENT APPLICATION

(11) **EP 3 991 765 A1**
(43) Date of publication of application: **04.05.2022**
(21) Application number: 20204324.6
(22) Date of filing: 28.10.2020
(51) Int. Cl.: A61M 1/16, A61M 1/28

(54) **APPARATUS FOR PUMPING AND DIALYSIS**

(71) Applicant: Bellco S.r.l., 41037 Mirandola (MO) (IT)
(72) Inventor: ORLANDINI, Salvatore, 41037 Modena (IT); MARRA, Antonia Giuseppe, 41037 Modena (IT); PETRUCCI, Alberto, 41037 Modena (IT)
(74) Representative: Maschio, Antonio

(57) **Abstract**

In medical apparatus, for example a dialysis machine, a pump in conjunction with a multichambered reservoir is provided. The volume pumped is determined by counting the number of fills of the reservoir made during a pumping phase. A less expensive pump may be used whilst maintaining an accurate determination of the volume pumped.

## Description

### FIELD OF THE INVENTION

This invention relates to medical apparatus in particular for dialysis and a pumping apparatus for use in such apparatus.

### BACKGROUND

One type of dialysis for the treatment of kidney failure is peritoneal dialysis (PD) which uses the lining of a patient's abdomen, the peritoneum, to filter blood while it remains inside the body. Peritoneal Dialysis is an effective way to treat patients with End Stage Renal Disease and acute patients with renal failure. The patient is provided with a catheter that includes a transfer set for selective connection to a dialysis solution or a drain bag. A dialysis solution ('dialysate' or 'peritoneal dialysis fluid') comprising water with other electrolytes is warmed to body temperature and then introduced into the peritoneal cavity of the patient via the catheter. The solution remains here for a period of time required to clean the blood of waste products, toxins and excess fluid and these are then removed from the body to leave the right amounts of electrolytes and nutrients in the blood. The machine or 'cycler' may repeat the exchange process a number of times (cycles), as required for a particular patient.

Conventionally dialysate consists of purified water, glucose and electrolytes, with the concentration of electrolytes resembling that which occurs naturally in the blood. It is prepared according to an individual patient's needs to help regulate their electrolyte and acid-base balance and remove metabolic waste products. The dialysate components are normally shipped in the form of concentrates, with basic and acidic concentrates being prepared separately. The basic concentrate is usually supplied as a dry concentrate consisting of pure sodium bicarbonate which is made up into a saturated solution and the acidic concentrate is usually provided as a liquid concentrate. If it is provided as a dry concentrate, the entire concentrate must be dissolved before being diluted to form the dialysate. This requires a large volume of liquid and multiple components for providing the peritoneal dialysis fluid, increasing the time required for production of the fluid. Concentrate may also be wasted during the production of the correct peritoneal dialysis fluid and difficulties may be encountered in providing the correct concentration of electrolytes in the fluid for a particular patient. In a newly developed dialysis machine it has been proposed to provide an architecture in which one part of the machine will be used to prepare the dialysate from purified water (purifying a domestic water supply) and the concentrate and another part to cycle the prepared dialysate through the patient via a catheter into the patent's peritoneum.

It will be appreciated that a suitable location for a water supply for generating the dialysate may not be conveniently located relative to the where the dialysis is to be performed. For example, the patient may prefer to rest on a bed in a bedroom and the nearest source of water may be located in a bathroom some metres distant.

In order to create the dialysate and to deliver it to the patient a pump is required. As will be understood, pumps for medical equipment need to be accurately controlled with high tolerances of manufacture and to be reliable. The volume of fluid pumped over any given time must predictable to ensure accurate control which means that the pumps are subject to stringent manufacturing tolerances. This makes such pumps expensive. Further, in use, a pump's tolerances may change through environmental factors, e.g. heat or cold or by wear from use. Accordingly, presently utilised pumps are expensive and have to be subject to regular maintenance and expensive replacement.

The invention arose in an attempt to mitigate or reduce these issues.

### SUMMARY OF THE INVENTION

According to the invention there is provided apparatus for pumping a fluid in medical equipment comprising a pump, having an inlet and an outlet, a reservoir divided into at least two chambers, means for selectively fluidly connecting the outlet of the pump to selectively one of the at least two chambers wherein each chamber is connected by means configured to selectively couple the chamber to a fluid outlet of the apparatus and a division for dividing the reservoir into the two chambers moveable to vary the volume of the chambers such that fluid being pumped into a first at least one of the at least two chambers increases the volume of the first of the at least one of the at least two chambers and decreases the volume of the at least one other of the at least two chambers by movement of the division.

Preferably, the division is provided by a moving wall. The moving wall may be provided by a piston moving within the reservoir acting as the dividing wall. However, most preferred is an arrangement in which the wall is formed by a flexible membrane or diaphragm.

Preferably, the flexible membrane is resiliently deformable. This is preferred because the diaphragm or membrane may be less expensive to produce than a piston and will be more easily formed to provide sealing engagement with the reservoir internal walls to provide a seal between the chambers.

In the described embodiment valves are provided to control the filling and emptying of the chambers. These are controlled by a processor to open and close in a synchronous manner.

In order to determine the volume pumped, the number of fills of the reservoir is determined. This is done by sensing when the division, membrane or diaphragm has moved by the desired predetermined amount. For example, sensors may be provided to determine when the membrane has reached the reservoir wall. Such sensors may be contact sensors, proximity sensors or optical sensors.

An aspect of the invention provides a dialysis machine comprising an apparatus for pumping as set out in the first aspect the fluid is then a dialysate although it will be appreciated that other fluids may be pumped by use of the apparatus.

In a further aspect of the invention there is provided apparatus for metering a fluid in medical equipment comprising providing one or more chambers each of a variable volume configured in use for connection to a pump for providing a fluid flow to one or more chambers, means to direct the flow into and out of the one or more chambers wherein the one or more chambers have a volume which is variable.

The volume of the chambers may be variable to cater for the performance of the pump to ensure that the volume of fluid delivered is controlled. Many ways of determining the volume may be applicable. However, preferably wherein the one or more chambers have a volume which is determined by a piston within a cylinder. This is preferred since it such arrangements are inexpensive to manufacture and provide reliable and accurate means for determining the volume.

Preferably, where there are two or more chambers are filled and emptied in a synchronous manner.

This may be done by a valve arrangement configured such that wherein when a first chamber is filled the other chamber is emptied.

The starting position of the piston within the cylinder may be adjusted in order to control the swept volume of the cylinder. Alternatively, or in addition the return position of the piston in the cylinder may be adjusted.

In the preferred embodiment, two pistons are linked by a common piston rod to be driven in a synchronous manner. This is driven by an actuator.

These aspects may be combined with a pump or configured to be attached to a pump by provision of a suitable fluid coupling.

In a broad aspect the invention provides a method of metering a fluid in medical equipment comprising the steps of; providing one or more metering chambers of adjustable volume, adjusting the volume of the one or more metering chambers to provide a desired volume and directing a fluid flow into and out of the one or more metering chambers and hence to an outlet..

### BRIEF DESCRIPTION OF THE FIGURES

A specific embodiment of the invention will now be described with reference to the figures in which:
Figure 1 shows in schematic form a dialysis machine in accordance with an embodiment of the invention in which a preparator module of the machine is located in a patient's bathroom and a cycler module of the machine is located in the patient's bedroom;
Figure 2 shows a pumping apparatus in accordance with the invention used in the dialysis machine of figure 1.
Figure 3 shows a further embodiment of the invention showing an active metering chambers during pumping phases.

### DETAILED DESCRIPTION

### Definitions

Unless defined otherwise, all technical and scientific terms used generally have the same meaning as commonly understood by one of ordinary skill in the art.

The articles "a" and "an" are used to refer to one or to over one (i.e., to at least one) of the grammatical object of the article. For example, "an element" means one element or over one element.

The term "comprising" includes, but is not limited to, whatever follows the word "comprising." Use of the term indicates the listed elements are required or mandatory but that other elements are optional and may be present.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "dialysate" describes a fluid into or out of which solutes from a fluid to be dialyzed diffuse through a membrane. An initial dialysate used for therapy typically contains electrolytes close in concentration to the physiological concentration of electrolytes found in blood. However, the concentration of the dialysate can change over the course of therapy, and can further be adjusted as desired.

The term "dialysis flow path" refers to a fluid pathway or passageway configured to convey a fluid, such as dialysate and/or blood, wherein said pathway forms at least part of, preferably the whole of, a fluid circuit for peritoneal dialysis, haemodialysis, hemofiltration, hemodiafiltration or ultrafiltration. A dialysis machine may be included within the flow path. A dialyzer may be included in the flow path.

The term "mix" means to combine one or more substance so that the resulting mixture is not easily separated. Mixing, especially evenly spreading, of solute and solvent aids and speeds the process of dissolution. Mixing can be achieved by any method, including spraying, stirring, shaking or otherwise agitating. The term "improved mixing" refers to a situation wherein the components of a mixture are more evenly distributed and not clumped together. Improved mixing may be achieved, for example, by speeding the mixing up. In the context of a solution, improved mixing results in a solution of the correct concentration because all or most of the solutes dissolve, rather than remaining as clumps or aggregated within the solvent.

The present invention relates to an improved automated peritoneal dialysis machine that includes a preparator and cycler for in situ preparation of peritoneal dialysis fluid (PDF) for delivery and drainage from a patient with communications links established therebetween.

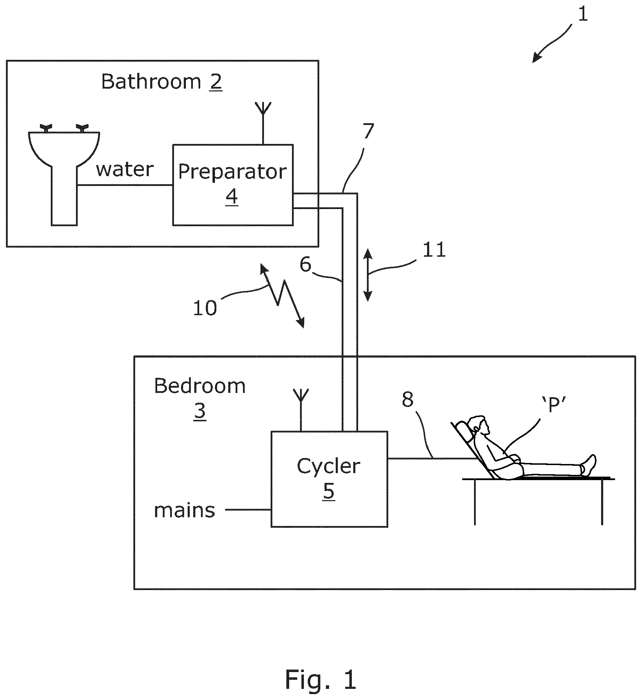

Fig. 1 shows an example of a peritoneal dialysis machine 1 according to the present invention. The peritoneal dialysis machine 1 is set up in the patient's home which is represented by two rooms, a bathroom 2 and an adjoining bedroom 3 (although these rooms are representative only and the locations could be other rooms). The peritoneal dialysis machine 1 includes a preparator 4, an automatic cycler 5, a flexible dialysate supply tube 6 and an electrical power supply line 7.

The preparator 4 is positioned in bathroom 2 as it requires a water source to enable the preparator 4 to generate peritoneal dialysis fluid. The water source may comprise, for example, a tap (faucet) or a more permanent outlet. The preparator 4 first filters the input water and mixes it with a quantity of powered dialysate in accordance with a preprogramed formulation. The powered dialysate will include dextrose, magnesium and calcium and lactate bicarbonate and sodium chloride

The automatic cycler 5 is positioned in the bedroom 3 where the patient "P" intends to undergo peritoneal dialysis. The cycler 5 is connected to a dialysate delivery tube 8 which is connected to a catheter (not shown) which is inserted into the peritoneum of the patient. The cycler 5 controls the flow of the dialysate into, and out of, the patient in accordance with the required dialysis procedure. Dialysate removed from the patient is stored in a container in the cycler 5 (not shown) for subsequent disposal. This procedure is predetermined and held in memory within the cycler 5 operated under processor control. Broadly, the machine operates by preparing dialysate at the preparator 4, the prepared dialysate is pumped via the dialysate supply tube 6 to the cycler 5. There it is heated to patient temperature and administered to the patent via the delivery tube 8 and the catheter into the patient. After, the appropriate time-period for dialysis, the dialysate is removed from the patient via catheter and the delivery tube 8 and held in storage for subsequent removal and disposal.

As will be appreciated, although the two modules are not collocated, it is necessary for instructions and data to pass between the modules. For example, the preparator 4 will need to provide status updates to the cycler 5 indicating that the dialysate is mixed and available for delivery. The cycler 5 will need to instruct the preparator 4 to commence with delivery, or to increase, or reduce the rate of delivery. Error states may need to be passed. For example, the preparator 4 may experience a problem with the water supply pressure and that will need to be communicated to the cycler and indicated as a fault condition on a display. Accordingly, the preparator 4 and the cycler 5 are each provided with a suitably programmed processor to control its respective functions. In addition, a master processor or controller is provided or designated to control the function of the machine as a whole. These processors (or one processor programmed to provide a number of processing functions) provide in addition a communication function to allow the modules to intemperate and to be controlled.

In this embodiment of the invention, there are two communications links which are operable to enable communications between the modules. A first communications link 10 using radio and operating in accordance with a Wi Fi communications protocol and a physical communications link 11 provided by cable 7 over which a Powerlink communications protocol is used.

In order to move the dialysate from the preparator 4 to the cycler 5 and to and from the patient, a pump or pumps are required. In this case the pump is located at the cycler 5 but it could be located at the preparator or indeed two or more pumps could be provided located at each module.

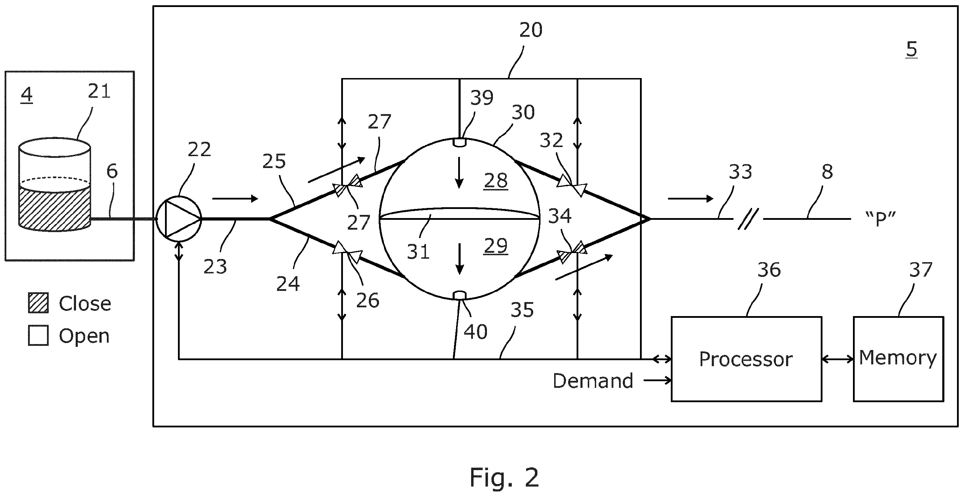

Figure 2 shows a pump apparatus 20 within the cycler 5. The pump apparatus 20 receives the dialysate from a mixing tank 21 located at the preparator 4 via supply tube 6 coupled to an inlet 22 to a rotary pump 22 which is electrically powered. The pump 22 has an outlet 23 which is coupled to a first and a second branch24, 25. Each branch includes a valve 26 and 27 which controls the flow into a respective chamber 28, 29 of a reservoir 30.

The reservoir 30 is defined by a generally spherical wall but is subdivided into the two chambers by an internally mounted diaphragm 31. The diaphragm is mounted at its peripheral edge to the interior surface of the reservoir wall. It is formed from an elastic plastics material which has a low modulus of elasticity. This means it is flexible and elastic to return to its central position but not highly resistant to impeded flow into or out of the respective chambers 28, 29.

The upper chamber 28 has an outlet which is fluidly coupled to a valve 32 and hence to a pump outlet 33. The lower chamber 29 has an outlet which is fluidly coupled to a valve 34 and hence to the outlet t 33. The outlet 33 is coupled to the delivery tube 8 and catheter to the patient "P".

The valves 32, 34, 26, 27 and pump 22 are coupled via a data and control bus 35 to a processor 36.

The processor 36 is a microprocessor which operates in accordance with a program held in memory 37 to control the way in which the apparatus pumps dialysate. It receives a demand signal on an input 38 and is responsive to pump the dialysate in the following manner.

The demand signal will indicate a volume of dialysate required for the patient treatment. This volume requirement will equate to a particular which will be met by a certain number of fills of the reservoir 30. The reservoir has a fixed and known volume which will not vary under operating conditions. This means that if the pump 22 has a performance which varies or is replaced by another pump of unknown performance following a fault it will not matter to the apparatus output since the demand is met by a certain number of reservoir fills.

Sensors are provided to determine when the reservoir is filled. A sensor 39 is located in the upper chamber and a second sensor is located in the lower chamber. These are linked to the processor 36 via the data and control bus 35. Many types of sensors may be used. For example, an optical , proximity sensor or contact sensor may be used which determines when the diaphragm 31 makes contact with the sensor. This will occur when the diaphragm is deflected and pushed by filling of the respective chambers.

Filling and emptying of the reservoir 30 will now be described. It occurs, in overview, as the pump 22 pumps fluid via one of the inlet valves 26, 27 into a respective chamber. To do this, one valve is opened and the other is closed. Simultaneously, the outlet valves are operated in a complimentary manner. This results in one chamber filling and the other emptying via the valve to the outlet 33.

The operation occurs in phases.

In a first phase, the lower chamber 29 is filled. This occurs when valve 26 is opened, valve 34 is closed. Dialysate is pumped into the chamber 29 pushing the diaphragm 31 upwards into chamber 28. This effectively results in chamber 28 being reduced in size and chamber 29 increasing in size. As will be understood in order for this to occur the valve 27 is closed and the valve 32 is opened. Any dialysate in chamber 28 is thus pushed out of chamber 28 as it is reduced in size by the diagram into the outlet 33. As the diaphragm makes contact with the upper part of the reservoir wall it will activate the sensor 39 and the processor 36 makes a count of one reservoir volume being pumped into the reservoir 39.

The processor in response to this advances the operation to the second phase.

In the second phase, valve 32 is closed and valve 27 is opened. Valve 26 is closed and valve 34 is opened. The pump 22 then pushes dialysate into the upper chamber 28, it fills and pushes the diaphragm downwards away from the upperpart of the reservoir wall and downwards towards the sensor 40. This results in the upper chamber 28 expanding in size as fluid is pushed via valve 34 to the outlet 33 and hence to the patient.

When the chamber 28 is fully filled, the sensor 40 is triggered by the diaphragm making contact and another volume increment is counted by the processor 36.

In the next cycle valve 34 is closed, valve 26 opened and valve 32 opened and valve 27 closed. Pumping then pushes the diaphragm upwards as chamber 29 fills with dialysate and fluid in the upper chamber 28 is passed via valve 32 to the outlet 33 as before.

The cycles or phases repeat until sufficient dialysate is pumped to the patient for treatment. In this manner the volume delivered to the patient is not dependent upon pump performance of pump 22 but by the reservoir size and the number of fills and required to provide the volume. This provides a more robust arrangement which may be used with a pump which is less expensive to produce than higher specification pumps.

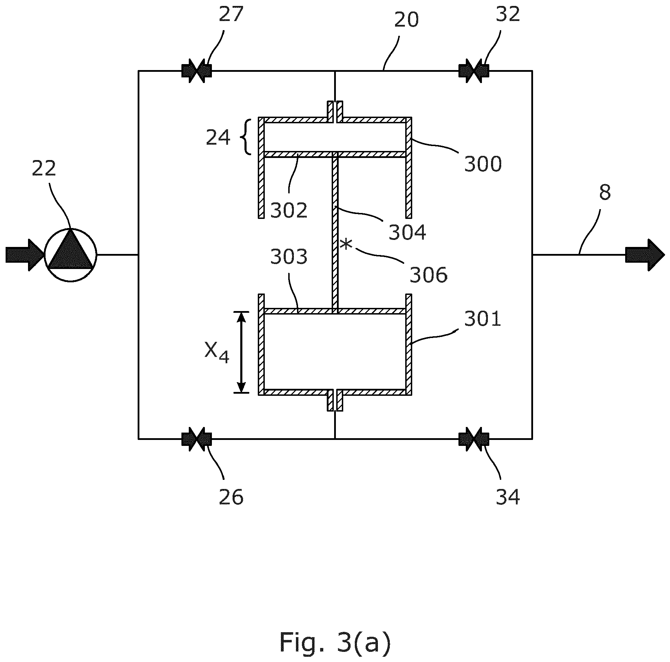

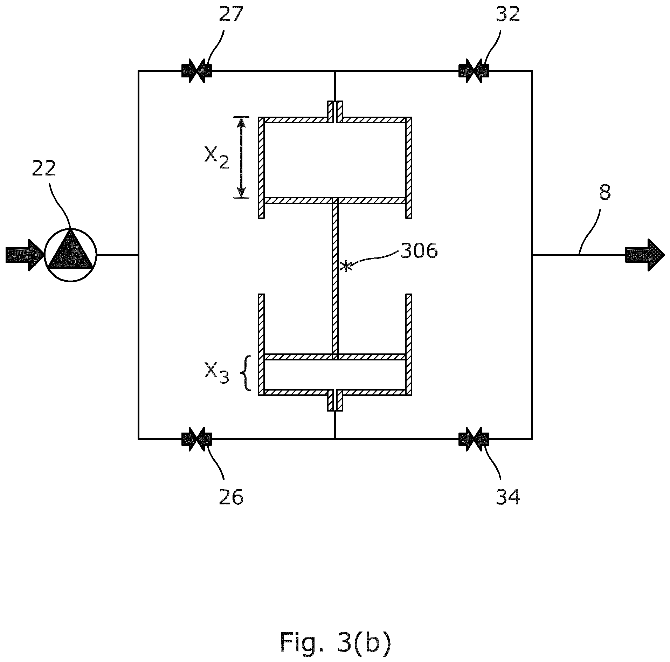

In alternative embodiments, the diaphragm may be replaced by a rigid wall moving in a manner of a piston. The reservoir may be made cylindrical or other shapes and the piston made in a complimentary cross-section to permit sealing movement within the cylinder. The diaphragm in the described embodiment, moves through the full extent of the vessel to make contact with the sensors. However, in alternatives, the diaphragm or piston may move only part of the way whilst being displaced to a known volume. Figure 3 shows an embodiment of the invention which illustrates this aspect.

As is shown in figure 3, in which like features bear the same reference numerals as the first embodiment. As before a pump 22 provides a fluid flow to be passed via a valve arrangement ,27, 26, 32 and 34 in and out of metering chambers 20. The valve opening and closing sequence is as before and is controlled in the same manner. In the first embodiment, the metering chamber is divided by a diaphragm. However, in this embodiment two chambers 300 and 301 are provided. These include a piston head 302 and 303 riding in respective cylinders and linked by a common piston rod 304. The piston rod 304 is driven by an actuator 306 and a rack and pawl gear arrangement. The actuator operates under microprocessor control to drive the pistons in a reciprocating manner. Figure 3a shows a first phase and 3b shows the opposite second phase.

The volume of the metering chambers is variable in accordance with the stroke of the piston which is set by the way in which the actuator is driven. In figure 3a we have an initial position. In this it will be seen that the upper piston 302 is located at a distance x1 from the top of the chamber. In the start of the opposite phase shown in figure 3b it will be seen that the piston head has reached a greater distances x2 form the top of the chamber. Thus, the swept volume in one phase will be determined from the relationship (x2-x1) multiplied by the area of the piston head and this is delivered to the output 8 in the next phase. The next phase will involve the emptying of this chamber 300 whilst the chamber 301 is filled. It will be seen form figure 3b that this chamber starts at the displacement x3 which in this embodiment will be equal to x1 because the pistons share a common piston rod. As the actuator 36 drives the piston rod, the piston head will move to the position shown in figure 3a to arrive at a displacement x4. Again, because the pistons share the same piston rod this will be equal to x2.

By controlling the actuator 306 to control the stroke, the volume delivered will be controlled. It is relatively easy to manufacture a cylinder of accurate known volume with an actuator of good performance. In this manner the pump 22 may be replaced or a lower specification pump may be used in the apparatus whilst the delivery of the fluid is accurately controlled.

Whilst in this embodiment, the chambers share a common piston rod, in alternative embodiments, they may be controlled independently by providing separate piston rods and or by driving the pistons over different swept volumes. That is to say, the embodiment of figure 3 may be driven such that the displacement varies between the phases, that is to say x1 may be different to x3 etc.

In alternative embodiments, the reservoirs or chambers may be divided into more than two chambers. This may offer an advantage in some situations where a more even pumping cycle is required.

In some embodiments, instead of a two metering chambers being provided, a single metering chamber may be provided.

One skilled in the art will understand that various combinations and/or modifications and variations can be made in the described apparatus, methods and uses depending upon the specific needs for operation. Moreover, features illustrated or described as being part of an aspect of the invention may be used in the aspect of the invention, either alone or in combination.

## Claims

1. Apparatus for pumping a fluid in medical equipment comprising a pump, having an inlet and an outlet, a reservoir divided into at least two chambers, means for selectively fluidly connecting the outlet of the pump to selectively one of the at least two chambers wherein each chamber is connected by means configured to selectively couple the chamber to a fluid outlet of the apparatus and a division for dividing the reservoir into the two chambers moveable to vary the volume of the chambers such that fluid being pumped into a first at least one of the at least two chambers increases the volume of the first of the at least one of the at least two chambers and decreases the volume of the at least one other of the at least two chambers by movement of the division.

2. Apparatus as claimed in claim 1, wherein the division is provided by a moving wall.

3. Apparatus as claimed in claim 2 wherein the wall is formed by a flexible membrane.

4. Apparatus as claimed in claim 3 wherein the flexible membrane is resiliently deformable.

5. Apparatus as claimed in any preceding claim wherein selectively operable valves are provided configured to control the filling and emptying of the chambers.

6. Apparatus as claimed in claim 5 wherein a first pair of valves is associated with a first chamber and and a second pair of valves is associated with a second chamber.

7. Apparatus as claimed in claim 6 wherein valves of the pairs are configured to open and close in a synchronised manner with valves of the other pair.

8. Apparatus as claimed in claim 7 wherein the valves operate under processor control.

9. Apparatus as claimed in claim 7 or 8, wherein the valves are configured such that a first valve of the first pair opens when a first valve of the second pair closes and a second valve of the first pair closes when a second valve of the second pair opens.

10. Apparatus as claimed in any preceding claim comprising sensors for providing an output for determining when the division has moved to a predetermined extent.

11. Apparatus as claimed in claim 10 wherein the sensors determine when the reservoir has been filled.

12. Apparatus as claimed in claim 11, wherein the sensors are located on an internal wall of the reservoir to determine when the division has made contact with or is within the proximity of the internal wall.

13. A dialysis machine comprising apparatus as claimed in any preceding claim wherein the fluid is a dialysate.

14. Apparatus for pumping a fluid in medical equipment comprising a pump, having an inlet and an outlet, a reservoir divided into at least two chambers, means for selectively fluidly connecting the outlet of the pump to selectively one of the at least two chambers wherein each chamber is connected by means configured to selectively couple the chamber to a fluid outlet of the apparatus and a division for dividing the reservoir into the two chambers moveable to vary the volume of the chambers such that fluid being pumped into a first at least one of the at least two chambers increases the volume of the first of the at least one of the at least two chambers and decreases the volume of the at least one other of the at least two chambers by movement of the division.

15. Apparatus for metering a fluid in medical equipment comprising providing one or more chambers each of a variable volume configured in use for connection to a pump for providing a fluid flow to one or more chambers, means to direct the flow into and out of the one or more chambers wherein the one or more chambers have a volume which is variable.

16. Apparatus as claimed in claim 15 wherein the one or more chambers have a volume which is determined by a piston within a cylinder.

17. Apparatus as claimed in claim 16 wherein respective pistons of respective chambers are operable in a synchronous manner.

18. Apparatus as claimed in claim 17 comprising a valve arrangement configured such that wherein when a first chamber is filled the other chamber is emptied.

19. Apparatus as claimed in any one of claims 16 to 18 wherein the swept volume of the piston within the chamber is variable.

20. Apparatus as claimed in claim 19 wherein two or more pistons are linked by a common piston rod to be driven in a synchronous manner.

21. Apparatus as claimed in claim 20 wherein the common piston rod is driven by an actuator.

22. Apparatus as claimed in any one of claims 14 to 21 in combination with a pump.

23. A method of metering a fluid in medical equipment comprising the steps of:
providing one or more metering chambers of adjustable volume;
adjusting the volume of the one or more metering chambers to provide a desired volume;
directing a fluid flow into and out of the one or more metering chambers.
